# FASCICULE DE BREVET EUROPEEN

(11) **EP 2 956 449 B1**
(45) Date de publication et mention de la délivrance du brevet: **28.06.2017**
(21) Numéro de dépôt: 14710599.3
(22) Date de dépôt: 14.02.2014
(51) Int. Cl.: C07D 309/06, C07D 335/02, C07D 309/10

(54) **PROCEDE DE SYNTHESE DE COMPOSES 4-(HETEROCYCLOALKYL)-BENZENE-1,3-DIOL**
VERFAHREN ZUR HERSTELLUNG 4-(HETEROCYCLOALKYL)BENZOL-1,3-DIOL-VERBINDUNGEN
PROCESS FOR THE PREPARATION OF 4-(HETEROCYCLOALKYL)-BENZENE-1,3-DIOL COMPOUNDS

(30) Priorité: 14.02.2013 FR 1351252; 14.02.2013 US 201361764638 P
(43) Date de publication de la demande: 23.12.2015
(73) Titulaire: Galderma Research & Development, 06410 Biot (FR)
(72) Inventeur: BOITEAU, Jean-Guy, F-06560 Valbonne (FR)
(74) Mandataire: Cabinet Becker et Associés
(86) Numéro de dépôt international: PCT/FR2014/050312
(87) Numéro de publication internationale: WO 2014/125231

(56) Documents cités:
- WO-A1-2010/063773
- NITTA A ET AL: "(3R)-3-Amino-4-(2,4,5-trifluorophenyl)-N- {4-[6-(2-methoxyethoxy)benzothiazol-2-yl]t etrahydropyran-4-yl}butanamide as a potent dipeptidyl peptidase IV inhibitor for the treatment of type 2 diabetes", BIOORGANIC & MEDICINAL CHEMISTRY LETTERS, PERGAMON, AMSTERDAM, NL, vol. 18, no. 20, 15 octobre 2008 (2008-10-15), pages 5435-5438, XP025562079, ISSN: 0960-894X, DOI: 10.1016/J.BMCL.2008.09.042 [extrait le 2008-09-12]

## Description

La présente invention se rapporte à un procédé de synthèse de composés 4-(hétérocycloalkyl)-benzène-1,3-diol répondant aux formules générales (I) et (II) suivantes : 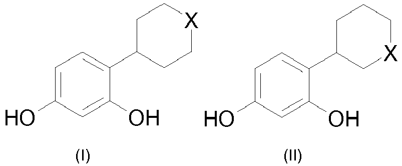 dans lesquelles X représente un atome d'oxygène ou un atome de soufre.

La synthèse de composés analogues à ceux des formules (I) et (II) ci-dessus a été décrite dans la demande de brevet WO 2010/063773. Cette synthèse est réalisée en trois étapes (figures 1 et 2)

Dans la première étape du procédé décrit dans la demande de brevet WO 2010/063773 (figures 1 et 2), le 2,4-dihydroxy bromobenzene (1) est mis en réaction avec du bromure de benzyle pour fournir le 2,4-dibenzyloxy bromobenzene (2).

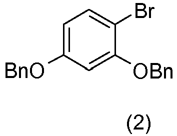

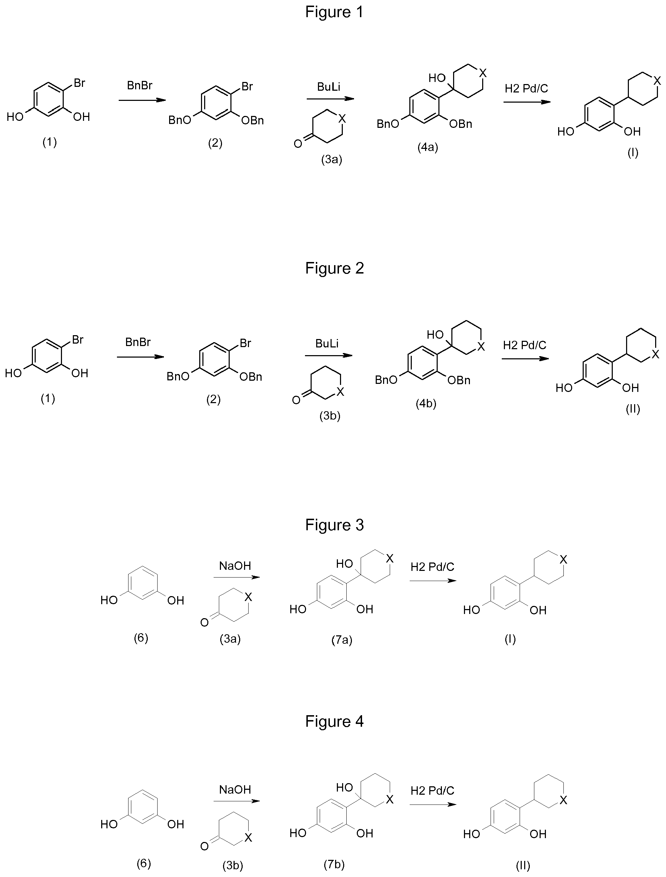

Le 2,4-dibenzyloxy bromobenzene (2) réagit ensuite en présence de butyllithium avec des hétérocycloalkanones de formule générale (3a) ou (3b) (Figures 1 et 2) pour fournir les alcools benzyliques, respectivement, de formule générale (4a) ou (4b).

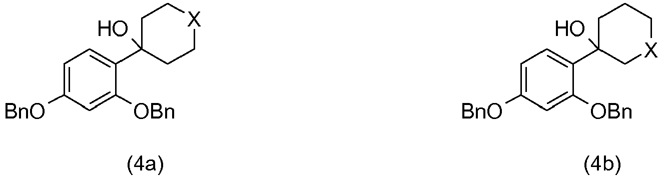

Les composés de formule générale (I) et (II) 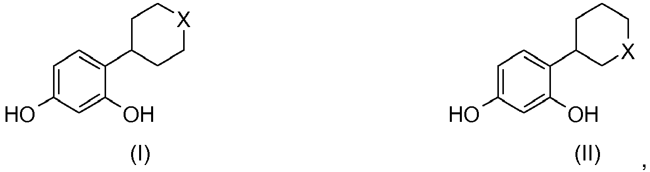 sont finalement obtenus, respectivement à partir des composés de formule générale (4a) et (4b), par hydrogénation dans le méthanol en présence d'hydrogène et d'un catalyseur à base de palladium.

Un premier inconvénient de ce procédé est l'introduction d'une séquence de réactions de protection / déprotection des phénols par des groupements benzyles. En effet, la nécessité de protéger les fonctions phénols du composé (1) conduit, d'une part, à rajouter deux étapes dans la synthèse des composés (I) et (II) et d'autre part, à augmenter inutilement la masse des intermédiaires (2), (4a) ou (4b), ce qui n'est pas adapté d'un point de vue industriel, notamment au niveau de l'économie d'atomes.

De plus, cette synthèse fait intervenir du bromure de benzyle dont les propriétés lacrymogènes rendent délicate la manipulation à l'échelle industrielle.

Un deuxième inconvénient de cette voie de synthèse est l'utilisation, à la deuxième étape, de conditions cryogéniques (Butyllithium, de l'ordre de -70°C) qui nécessitent un équipement adapté à l'échelle industrielle et qui ont pour conséquence un coût supplémentaire dans ce procédé.

Enfin, un troisième inconvénient de ce procédé est le prix élevé du 2,4-dihydroxy bromobenzene ainsi que celui du butyllithium.

Dans le cadre du développement de ces composés, il existe un besoin de disposer d'une méthode permettant de préparer économiquement et dans des conditions de sécurité les composés de formule générale (I) ou (II) tout en évitant les inconvénients mentionnés ci-dessus.

La présente invention vise donc à résoudre les problèmes cités ci-dessus en proposant un procédé de synthèse de 4-(hétérocycloalkyl)-benzene-1,3-diol de formule générale (I) ou (II), comportant moins d'étapes, plus économique, plus simple et susceptible d'être adapté à l'échelle industrielle.

L'objet de la présente invention est relatif à un procédé de synthèse d'un 4-(hétérocycloalkyl)-benzene-1,3-diol répondant à la formule générale (I) 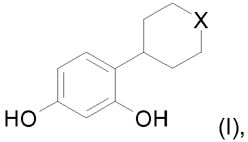 dans laquelle X représente un atome d'oxygène ou de soufre, caractérisé en ce qu'un composé répondant à la formule générale (7a) 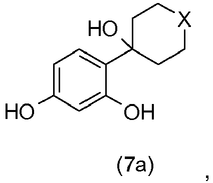 réagit avec de l'hydrogène en présence d'un catalyseur à base de palladium dans un solvant polaire lorsque X représente un atome d'oxygène ou avec un réducteur de type hydrure de silicium et un acide de Lewis dans un solvant apolaire lorsque X représente un atome de soufre. De préférence, X représente un atome d'oxygène.

Un autre objet de l'invention concerne un procédé de synthèse d'un 4-(hétérocycloalkyl)-benzene-1,3-diol répondant à la formule générale (II) 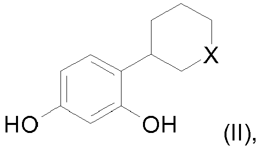 dans laquelle X représente un atome d'oxygène ou de soufre, caractérisé en ce qu'un composé répondant à la formule générale (7b) 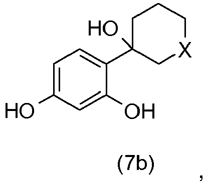 réagit avec de l'hydrogène en présence d'un catalyseur à base de palladium dans un solvant polaire lorsque X représente un atome d'oxygène ou avec un réducteur de type hydrure de silicium et un acide de Lewis dans un solvant apolaire lorsque X représente un atome de soufre.

De préférence, le solvant polaire utilisé dans les deux procédés ci-dessus est choisi dans le groupe comprenant les alcools comme le méthanol par exemple, les acides carboxyliques comme l'acide acétique par exemple, les esters comme l'acétate d'éthyle par exemple, les éthers comme le tetrahydrofuranne par exemple, l'eau et un mélange de ces solvants. Avantageusement, les alcools sont choisis parmi le méthanol, l'éthanol et l'isopropanol.

De préférence, le solvant apolaire est choisi dans le groupe comprenant le dichlorométhane et le dichloro-1,2-éthane.

De préférence, le catalyseur à base palladium est choisi dans le groupe constitué par le palladium sur charbon, l'hydroxyde de palladium et l'acétate de palladium.

De préférence, la pression d'hydrogène utilisée dans les procédés de l'invention est comprise entre 1 et 10 bars.

Dans un autre mode de réalisation particulier, le composé répondant à la formule générale (7a) 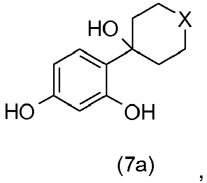 dans laquelle X représente un atome d'oxygène ou un atome de soufre est obtenu par réaction du résorcinol avec une heterocycloalkanone répondant à la formule générale (3a) 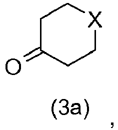 dans un solvant polaire et en présence d'une base.

Dans un autre mode de réalisation particulier, le composé répondant à la formule générale (7b) 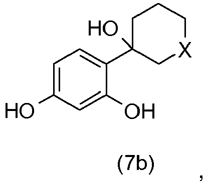 dans laquelle X représente un atome d'oxygène ou un atome de soufre est obtenu par réaction du résorcinol avec une heterocycloalkanone répondant à la formule générale (3b) 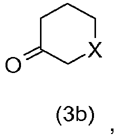 dans un solvant polaire et en présence d'un base.

De préférence, la base est choisie dans le groupe comprenant l'hydroxyde de sodium, l'hydroxyde de potassium, l'hydroxyde de lithium et les alcoolates de métaux, de préférence le méthanolate de sodium et le tert-butylate de potassium. Avantageusement, la base est l'hydroxyde de sodium ou l'hydroxyde de potassium.

De préférence, le solvant polaire est choisi dans le groupe comprenant l'eau et les alcools, de préférence le méthanol, l'éthanol, et l'isopropanol. Avantageusement, le solvant polaire est l'eau.

De préférence, le résorcinol et l'hétérocycloalkanone (3a) ou (3b) sont utilisés dans un rapport molaire résorcinol/hétérocycloalkanone compris entre 1 et 8.

Un autre objet de l'invention concerne le composé de formule générale (7a) ou (7b) 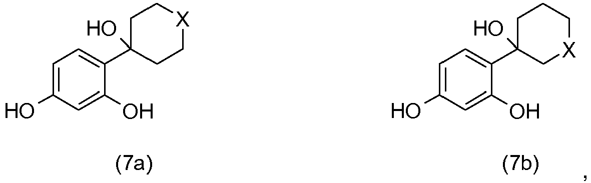 dans laquelle X représente un atome d'oxygène ou un atome de soufre. De préférence le composé est choisi dans le groupe constitué par le 4-(4-Hydroxy-tetrahydro-pyran-4-yl)-benzene-1,3-diol et le 4-(3-Hydroxy-tetrahydro-pyran-3-yl)-benzene-1,3-diol.

Le nouveau procédé de synthèse de 4-(hétérocycloalkyl)-benzene-1,3-diol (I) ou (II), de l'invention et tel qu'illustré aux figures 3 et 4, présente l'avantage d'être une synthèse courte comprenant au maximum une séquence linéaire de 2 étapes.

Cette nouvelle voie de synthèse utilise le résorcinol (6) comme produit de départ, ce qui, économiquement, est très avantageux comparé au prix du 2,4-dihydroxy bromobenzene (1).

La première étape de cette nouvelle voie de synthèse est une étape originale de couplage du résorcinol (6) avec les hétérocycloalkanones (3a) ou (3b) en présence d'une base. La base utilisée est choisie de préférence dans le groupe comprenant l'hydroxyde de sodium, l'hydroxyde de potassium, l'hydroxyde de lithium et les alcoolates de métaux comme le méthanolate de sodium ou le tert-butylate de potassium par exemple. Cette étape est réalisée dans un solvant polaire, de préférence dans l'eau ou dans des alcools, et permet d'obtenir directement les composés (7a) ou (7b) respectivement. Les alcools sont choisis de préférence dans le groupe comprenant le méthanol, l'éthanol et l'isopropanol.

Lors de cette première étape, un excès de résorcinol (6) est de préférence utilisé pour réagir sur l'hétérocycloalkanone (3a) ou (3b). Plus spécifiquement, le rapport molaire résorcinol/hétérocycloalkanone est compris entre 1 et 8 et, de préférence, entre 2 et 4.

La température à laquelle est effectuée cette étape de couplage représente un autre avantage de cette nouvelle voie de synthèse. En effet, cette première étape est réalisée à température ambiante, dans l'eau ou dans les alcools, ce qui permet d'éviter l'étape cryogénique (de l'ordre de -70°C) du procédé divulgué dans WO 2010/063773. D'autre part, les intermédiaires réactionnels (7a) ou (7b) sont obtenus sous forme cristalline après neutralisation du milieu et permet de s'affranchir des étapes de purification. Ainsi, la séparation sur une colonne de chromatographie pour isoler les intermédiaires réactionnels (7a) ou (7b) 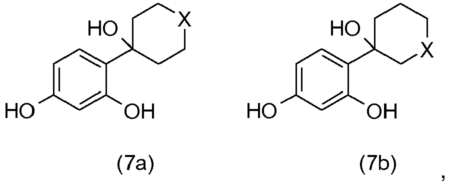 n'est pas nécessaire, ce qui constitue un autre avantage pour la transposition de ce procédé à une échelle industrielle. Il est préférable d'avoir un pH égal à 7 ou un pH de 7 à 8. De manière préférentielle le pH est égal à 7.

La seconde étape de cette nouvelle voie de synthèse est une étape d'hydrogénation ou de réduction des intermédiaires (7a) ou (7b) pour obtenir respectivement les composés de formule (I) ou (II).

Dans le cas où X représente un atome d'oxygène, l'hydrogénation des intermédiaires (7a) ou (7b) est réalisée dans un solvant polaire. Les solvants polaires préférés sont choisis dans le groupe comprenant les alcools comme le méthanol par exemple, les acides carboxyliques comme l'acide acétique par exemple, les esters comme l'acétate d'éthyle par exemple, les éthers comme le tetrahydrofuranne par exemple, l'eau et un mélange de ces solvants. Les alcools sont choisis de préférence dans le groupe comprenant le méthanol, l'éthanol et l'isopropanol.

L'hydrogénation est réalisée en présence d'un catalyseur à base de palladium. Les catalyseurs préférés sont choisis dans le groupe constitué par le palladium sur charbon, l'hydroxyde de palladium, l'acétate de palladium, ou encore tout autre catalyseur de réduction connu de l'homme du métier. La pression d'hydrogène utilisée est comprise entre 1 et 10 bars et de préférence entre 3 et 7 bars.

Dans le cas où X représente un atome de soufre, la réduction des intermédiaires (7a) ou (7b) est réalisée dans un solvant apolaire. Les solvants apolaires préférés sont choisis dans le groupe comprenant le dichlorométhane et le dichloro-1,2-éthane. La réduction est réalisée en présence d'un réducteur de type hydrure de silicium et d'un acide de Lewis. Les réducteurs préférés sont choisis dans le groupe constitué par le triethylsilane, le polymethylhydrosiloxane (PMHS) et encore tout autre hydrure de silicium connu de l'homme du métier. Les acides de Lewis préférés sont choisis dans le groupe constitué par le trifluorure de bore et ses complexes comme par exemple l'éthérate de trifluoroborane.

### Description des Figures

Figure 1 : Préparation des composés de formule (I) décrite dans WO 2010/063773
Figure 2 : Préparation des composés de formule (II) décrite dans WO 2010/063773
Figure 3: Procédé de préparation des composés de l'invention de formule (I)
Figure 4: Procédé de préparation des composés de l'invention de formule (II)

### Exemples

Les exemples suivants sont maintenant présentés en vue d'illustrer le procédé tel que décrit précédemment. Ces exemples qui illustrent le procédé de l'invention ne sont pas limitatifs.

### Exemple 1 : cas X = O : 4-(Tetrahydro-pyran-4-yl)-benzene-1,3-diol

### a) 4-(4-Hydroxy-tetrahydro-pyran-4-yl)-benzene-1,3-diol

Dans un réacteur double enveloppe de 6 L, 550 g (5 moles, 4eq) de résorcinol sont chargés sous azote puis 1530 ml d'une solution de soude 3M sont ajoutés en maintenant la température à 17°C. 125 g de tetrahydro-4-pyranon-4-one (1.25 mol, 1 eq) sont ajoutés au milieu réactionnel. Le mélange est alors agité 2 heures à température ambiante, l'avancement de la réaction est contrôlé par plaque CCM (éluant hept /AcOEt 2/1). La réaction est arrêtée par l'ajout d'une solution d'acide chlorhydrique 2M jusqu'à obtenir un pH = 7 (pH metre). 830 g de NaCl sont alors ajoutés et le milieu réactionnel est agité une nuit à température ambiante. Le solide est filtré puis lavé trois fois avec 11 d'eau. Le solide est essoré puis séché sous vide à 50°C une nuit pour fournir 125 g de 4-(4-hydroxy-tetrahydro-pyran-4-yl)-benzene-1,3-diol sous forme de poudre blanche cristalline. Rdt = 48%. Tf = 152 ° C
**RMN ¹H (DMSO D6, 400 MHz):** 1.50 (m, 2H) ; 2.21 (m, 2H) ; 3.69 (m, 4H) ; 5.50 (sl, 1 H) ; 6.20 (m, 2H) ; 7.16 (d, *J* = 8 Hz, 1 H) ; 6.82 (d, *J* = 8.4 Hz, 1 H) ; 8.95 (s, 1 H) ; 9.11 (sl, 2H).

### b) 4-(Tetrahydro-pyran-4-yl)-benzene-1,3-diol

Dans un réacteur de PARR sous azote, 100 g (0.47 mol) de 4-(4-hydroxy-tetrahydro-pyran-4-yl)-benzene-1,3-diol sont placés suivis par 100 mL d'acide acétique ainsi que 10 g de palladium sur charbon à 10%. 400 mL de THF sont ajoutés et le milieu réactionnel est agité 5 heures sous 5 bars d'hydrogène et à température ambiante. La solution est alors filtrée sur clarcel, le clarcel est lavé avec 250 ml de THF puis le filtrat est concentré jusqu'à obtenir un volume final de150 mL. 50 mL d'acétate d'éthyle sont alors ajoutés et le mélange réactionnel est agité une heure à 0°C. Le solide est filtré puis lavé avec 50 ml d'acétate d'éthyle supplémentaire puis séché sous vide pour fournir 84 g de 4-(Tetrahydro-pyran-4-yl)-benzene-1,3-diol sous forme de produit cristallin. Rdt = 92%.
Tf = 223 °C
**RMN ¹H (DMSO D6, 400 MHz):** 1.54 (m, 4H) ; 2.92 (m, 1 H) ; 3.39 (m, 2H) ; 3.90 (m, 2H) ; 6.14 (dd, *J* = 8.4 & 2.4 Hz, 1 H) ; 6.25 (d, *J* = 2.4 Hz, 1 H) ; 6.82 (d, *J* = 8.4 Hz, 1 H) ; 8.95 (s, 1 H) ; 9.11 (s, 1 H).
**RMN ¹³C (DMSO D6, 100 MHz)** : 32.6, 33.5, 67.7, 102.3, 106.0, 122.4, 126.7, 155.2, 156.0.

### Exemple 2: cas X = S : 4-(Tetrahydro-thiopyran-4-yl)-benzene-1,3-diol

### a) 4-(4-hydroxy-tetrahydro-thiopyran-4-yl)-benzene-1,3-diol

13.43 g (0.115 mol, 1 éq) de tétrahydro-thiopyran-4-one en suspension dans 100 mL d'eau sont ajoutés sur un mélange dégazé de 25.45 g (0.231 mol, 2.0 éq) de résorcinol solubilisés dans 115 mL (0.231 mol, 2.0 éq) d'hydroxyde de sodium 2N. Le milieu réactionnel est agité à température ambiante pendant 2 heures et 30 minutes. Le milieu réactionnel est traité avec 100 mL d'une solution d'acide chlorhydrique 2N (pH 5) et extrait à l'acétate d'éthyle. Les phases organiques sont rassemblées, séchées sur sulfate de magnésium, filtrées et évaporées. 40.88 g d'huile jaune sont obtenus.
Cette huile est solubilisée dans 1 L de dichlorométhane et engagée dans l'étape suivante.

### b) 4-(Tétrahydro-thiopyran-4-yl)-benzène-1,3-diol

55 mL (0.345 mol, 3 éq) de triéthylsilane suivis de 44 mL (0.345 mol, 3 éq) d'éthérate de trifluoroborane sont ajoutés goutte à goutte sur une solution (0.115 mol, 1 éq) de 4-(4-hydroxy-tetrahydro-thiopyran-4-yl)-benzene-1,3-diol brut tel qu'obtenu à l'étape précédente dans 1 L de dichlorométhane. Le milieu réactionnel est agité à température ambiante pendant 10 minutes. 400 mL d'eau sont ajoutés dans le milieu réactionnel suivis de 250 mL d'une solution saturée d'hydrogénocarbonate de sodium. Le mélange est décanté et la phase aqueuse est extraite avec du dichlorométhane. Les phases organiques sont rassemblées, lavées à l'eau, séchées sur sulfate de magnésium et concentrées. L'insoluble est filtré. 6.85g de 4-(tétrahydro-thiopyran-4-yl)-benzène-1,3-diol sont obtenus sous forme d'une poudre blanche. Rendement = 28% sur les deux étapes
Tf = 171 °C
**RMN ¹H (DMSO D6, 400 MHz):** 1.61 (m, 2H) ; 1.92 (m, 2H) ; 2.55 (m, 2H), 2.72 (m, 3H) ; 6.14 (dd, *J* = 8.4 & 2.4 Hz, 1 H) ; 6.25 (d, *J* = 2.4 Hz, 1 H) ; 6.80 (d, *J* = 8.4 Hz, 1 H) ; 8.95 (s, 1 H) ; 9.10 (s, 1 H).
**RMN ¹³C (DMSO D6, 100 MHz)** : 28.7, 33.8, 35.6, 102.3, 106.0, 123.5, 126.7, 154.8, 156.0.

### Exemple 3 : cas X = O : 4-(3-Hydroxy-tétrahydro-pyran-3-yl)-benzène-1,3-diol

### a) 4-(3-hydroxy-tétrahydro-pyran-3-yl)-benzène-1,3-diol

2.1 g (0.02 mol, 1 éq) de dihydro-pyran-3-one en solution dans 2 mL d'eau sont ajoutés goutte à goutte sur un mélange dégazé de 8.8 g (0.08 mol, 4.0 éq) de résorcinol solubilisés dans 25 mL (0.075 mol, 3.7 éq) d'hydroxyde de sodium 3N. Le milieu réactionnel est agité à température ambiante pendant 1 heure. Le milieu réactionnel est traité avec 5 mL d'une solution d'acide chlorhydrique concentré (pH 7) et 9 g de chlorure de sodium sont ajoutés. Le mélange est refroidi dans un bain de glace et extrait à l'acétate d'éthyle. Les phases organiques sont rassemblées, séchées sur sulfate de magnésium, filtrées et évaporées. Le résidu est chromatographié sur gel de silice (colonne AnaLogix SF40-150g, Spot II) élué à l'heptane/acétate d'éthyle 80/20 puis 20/80. 2.91 g de 4-(3-hydroxy-tétrahydro-pyran-3-yl)-benzène-1,3-diol sont obtenus sous forme d'un solide amorphe blanc. Rendement = 69%

### b) 4-(Tétrahydro-pyran-3-yl)-benzène-1,3-diol

Un mélange de 2.9 g (0.14 mol, 1 éq) de 4-(3-hydroxy-tétrahydro-pyran-3-yl)-benzène-1,3-diol dans 60 mL d'acétate d'éthyle et 6 mL de méthanol en présence de 580 mg (20% en masse) de palladium sur charbon à 10% est agité sous pression d'hydrogène de 5 bars à température ambiante pendant 5 heures puis chauffé à 50°C pendant 24 heures (formation de 10% de produit). 580 mg (20% en masse) de palladium sur charbon à 10% sont rajoutés et le milieu réactionnel est chauffé à 60°C sous pression d'hydrogène de 5 bars pendant 6 jours. 580 mg (20% en masse) de palladium sur charbon à 10% sont rajoutés et le milieu réactionnel est chauffé à 60°C sous pression d'hydrogène de 5 bars pendant 24 heures. Le milieu réactionnel est filtré sur papier filtre et le filtrat est évaporé. Le résidu est chromatographié sur gel de silice (colonne AnaLogix SF40-150g, Spot II) élué à l'heptane/acétate d'éthyle 78/22 à 50/50. L'huile obtenue est cristallisée dans dichlorométhane/heptane, filtré et séché sous vide à 40°C. 1.21 g de 4-(tétrahydro-pyran-3-yl)-benzène-1,3-diol sont obtenus sous forme d'un solide blanc. (Tf = 148-149°C). Rendement = 44%
**RMN ¹H (DMSO D6, 400 MHz):** 1.54-1.77 (m, 4H) ; 2.94 (m, 1 H) ; 3.12 (t, *J* = 10.6 Hz, 1H) ; 3.30 (m, 1H), 3.75 (m, 1H) ; 3.81 (d, *J* = 11 Hz, 1 H); 6.15 (dd, *J* = 8.4 & 2.4 Hz, 1 H) ; 6.26 (d, *J* = 2.4 Hz, 1 H); 6.84 (d, *J* = 8.4 Hz, 1 H) ; 9.00 (s, 1 H) ; 9.18 (s, 1 H).
**RMN ¹³C (DMSO D6, 100 MHz)** : 26.2; 28.8; 34.9; 67.2; 72.1; 102.3; 106.0; 119.0; 127.3; 155.6; 156.4.

## Revendications

1. Procédé de synthèse d'un 4-(hétérocycloalkyl)-benzene-1,3-diol répondant à la formule générale (I) 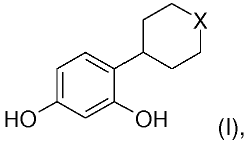 dans laquelle X représente un atome d'oxygène ou de soufre, **caractérisé en ce que** un composé répondant à la formule générale (7a) 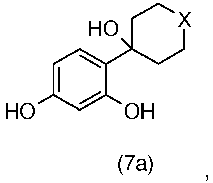 réagit avec de l'hydrogène en présence d'un catalyseur à base de palladium dans un solvant polaire lorsque X représente un atome d'oxygène ou avec un réducteur de type hydrure de silicium et un acide de Lewis dans un solvant apolaire lorsque X représente un atome de soufre.

2. Procédé de synthèse d'un 4-(heterocycloalkyl)-benzene-1,3-diol répondant à la formule générale (II) 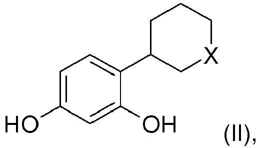 dans laquelle X représente un atome d'oxygène ou de soufre, **caractérisé en ce que** un composé répondant à la formule générale (7b) 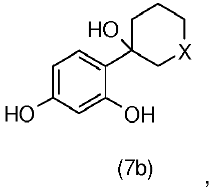 réagit avec de l'hydrogène en présence d'un catalyseur à base de palladium dans un solvant polaire lorsque X représente un atome d'oxygène ou avec un réducteur de type hydrure de silicium et un acide de Lewis dans un solvant apolaire lorsque X représente un atome de soufre.

3. Procédé selon la revendication 1 ou 2, **caractérisé en ce que** le solvant polaire est choisi dans le groupe comprenant les alcools, de préférence le méthanol, les acides carboxyliques, de préférence l'acide acétique, les esters, de préférence l'acétate d'éthyle, les éthers, de préférence le tetrahydrofuranne, l'eau et un mélange de ces solvants.

4. Procédé selon la revendication 3, **caractérisé en ce que** les alcools sont choisis dans le groupe comprenant le méthanol, l'éthanol et l'isopropanol.

5. Procédé selon la revendication 1 ou 2, **caractérisé en ce que** le solvant apolaire est choisi dans le groupe comprenant le dichlorométhane et le dichloro-1,2-éthane.

6. Procédé selon la revendication 1 ou 2, **caractérisé en ce que** le catalyseur à base de palladium est choisi dans le groupe constitué par le palladium sur charbon, l'hydroxyde de palladium et l'acétate de palladium.

7. Procédé selon la revendication 1 ou 2, **caractérisé en ce que** la pression d'hydrogène utilisée est comprise entre 1 et 10 bars.

8. Procédé selon la revendication 1, **caractérisé en ce que** le composé répondant à la formule générale (7a) 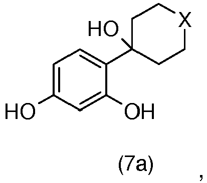 dans laquelle X représente un atome d'oxygène ou un atome de soufre est obtenu par réaction du résorcinol avec une heterocycloalkanone répondant à la formule générale (3a) 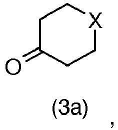 dans un solvant polaire et en présence d'une base.

9. Procédé selon la revendication 2, **caractérisé en ce que** le composé répondant à la formule générale (7b) 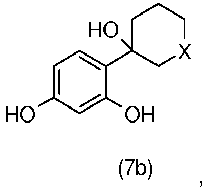 dans laquelle X représente un atome d'oxygène ou un atome de soufre est obtenu par réaction du résorcinol avec une heterocycloalkanone répondant à la formule générale (3b) 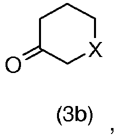 dans un solvant polaire et en présence d'un base.

10. Procédé selon la revendication 8 ou 9, **caractérisé en ce que** la base utilisée est choisie dans le groupe comprenant l'hydroxyde de sodium, l'hydroxyde de potassium, l'hydroxyde de lithium et les alcoolates de métaux comme le methanolate de sodium ou le tert-butylate de potassium, et de préférence choisie dans le groupe comprenant l'hydroxyde de sodium et l'hydroxyde de potassium.

11. Procédé selon la revendication 8 ou 9, **caractérisé en ce que** solvant polaire est choisi dans le groupe comprenant l'eau et les alcools, de préférence, l'eau, le méthanol, l'éthanol ou l'isopropanol.

12. Procédé selon la revendication 8 ou 9, **caractérisé en ce que** le résorcinol et l'heterocycloalkanone (3a) ou (3b) sont utilisés dans un rapport molaire résorcinol/heterocycloalkanone compris entre 1 et 8.

13. Composé de formule générale (7a) ou (7b) 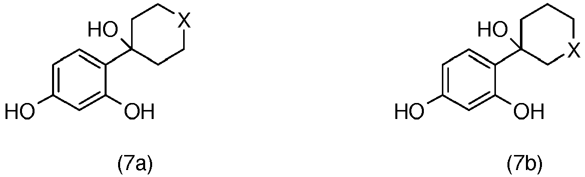 dans laquelle X représente un atome d'oxygène ou un atome de soufre.

14. Composé selon la revendication 13 **caractérisé en ce qu'**il est choisi dans le groupe constitué par le 4-(4-Hydroxy-tetrahydro-pyran-4-yl)-benzene-1,3-diol et le 4-(3-Hydroxy-tetrahydro-pyran-3-yl)-benzene-1,3-diol.

15. Procédé de synthèse selon la revendication 1 **caractérisé en ce que** X représente un atome d'oxygène.

## Patentansprüche

1. Verfahren zur Synthese eines 4-(Heterocycloalkyl)-benzol-1,3-diols mit der allgemeinen Formel (I) 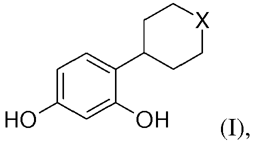 in der X für ein Sauerstoff- oder Schwefelatom steht, **dadurch gekennzeichnet, dass** eine Verbindung mit der allgemeinen Formel (7a) 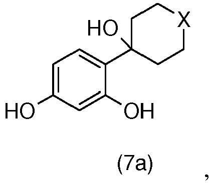 mit Wasserstoff in Gegenwart eines Katalysators auf Palladium-Basis in einem polaren Lösemittel, wenn X für ein Sauerstoffatom steht, oder mit einem Reduktionsmittel vom Typ Siliciumwasserstoff und einer Lewis-Säure in einem apolaren Lösemittel, wenn X für ein Schwefelatom steht, umgesetzt wird.

2. Verfahren zur Synthese eines 4-(Heterocycloalkyl)-benzol-1,3-diols mit der allgemeinen Formel (II) 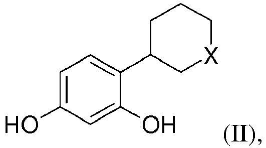 in der X für ein Sauerstoff- oder Schwefelatom steht, **dadurch gekennzeichnet, dass** eine Verbindung mit der allgemeinen Formel (7b) 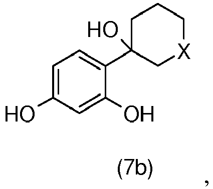 mit Wasserstoff in Gegenwart eines Katalysators auf Palladium-Basis in einem polaren Lösemittel, wenn X für ein Sauerstoffatom steht, oder mit einem Reduktionsmittel vom Typ Siliciumwasserstoff und einer Lewis-Säure in einem apolaren Lösemittel, wenn X für ein Schwefelatom steht, umgesetzt wird.

3. Verfahren gemäß Anspruch 1 oder 2, **dadurch gekennzeichnet, dass** das polare Lösemittel aus der Gruppe ausgewählt ist, umfassend Alkohole, vorzugsweise Methanol, Carbonsäuren, vorzugsweise Essigsäure, Ester, vorzugsweise Ethylacetat, Ether, vorzugsweise Tetrahydrofuran, Wasser und ein Gemisch dieser Lösemittel.

4. Verfahren gemäß Anspruch 3, **dadurch gekennzeichnet, dass** die Alkohole aus der Gruppe ausgewählt sind, umfassend Methanol, Ethanol und Isopropanol.

5. Verfahren gemäß Anspruch 1 oder 2, **dadurch gekennzeichnet, dass** das apolare Lösemittel aus der Gruppe ausgewählt ist, umfassend Dichlormethan und 1,2-Dichlorethan.

6. Verfahren gemäß, Anspruch 1 oder 2, **dadurch gekennzeichnet, dass** der Katalysator auf Palladium-Basis aus der Gruppe ausgewählt ist, bestehend aus Palladium auf Kohle, Palladiumhydroxid und Palladiumacetat.

7. Verfahren gemäß Anspruch 1 oder 2, **dadurch gekennzeichnet, dass** der angewandte Wasserstoffdruck zwischen 1 und 10 bar beträgt.

8. Verfahren gemäß Anspruch 1, **dadurch gekennzeichnet, dass** die Verbindung mit der allgemeinen Formel (7a) 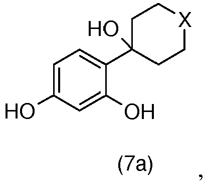 in der X für ein Sauerstoffatom oder ein Schwefelatom steht, durch Reaktion von Resorcin mit einem Heterocycloalkanon mit der allgemeinen Formel (3a) 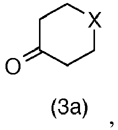 in einem polaren Lösemittel und in Gegenwart einer Base erhalten wird.

9. Verfahren gemäß Anspruch 2, **dadurch gekennzeichnet, dass** die Verbindung mit der allgemeinen Formel (7b) 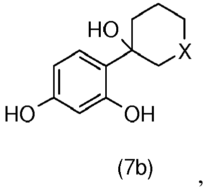 in der X für ein Sauerstoffatom oder ein Schwefelatom steht, durch Reaktion von Resorcin mit einem Heterocycloalkanon mit der allgemeinen Formel (3b) 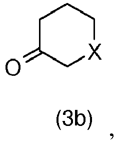 in einem polaren Lösemittel und in Gegenwart einer Base erhalten wird.

10. Verfahren gemäß Anspruch 8 oder 9, **dadurch gekennzeichnet, dass** die eingesetzte Base aus der Gruppe ausgewählt ist, umfassend Natriumhydroxid, Kaliumhydroxid, Lithiumhydroxid und Metallalkoholate wie Natriummethoxid oder Kalium-tert.-butylat, und vorzugsweise aus der Gruppe ausgewählt ist, umfassend Natriumhydroxid und Kaliumhydroxid.

11. Verfahren gemäß Anspruch 8 oder 9, **dadurch gekennzeichnet, dass** das polare Lösemittel aus der Gruppe ausgewählt ist, umfassend Wasser und Alkohole, vorzugsweise Wasser, Methanol, Ethanol oder Isopropanol.

12. Verfahren gemäß Anspruch 8 oder 9, **dadurch gekennzeichnet, dass** Resorcin und der Heterocycloalkanon (3a) oder (3b) in einem Resorcin/Heterocycloalkanon-Molverhältnis zwischen 1 und 8 eingesetzt werden.

13. Verbindung der allgemeinen Formel (7a) oder (7b) 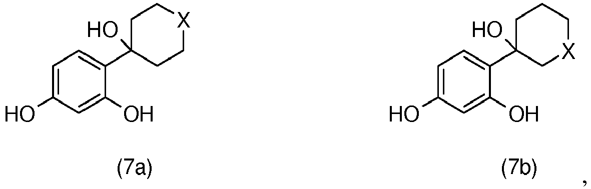 in der X für ein Sauerstoffatom oder ein Schwefelatom steht.

14. Verbindung gemäß Anspruch 13, **dadurch gekennzeichnet, dass** sie aus der Gruppe ausgewählt ist, bestehend aus 4-(4-Hydroxytetrahydropyran-4-yl)-benzol-1,3-diol und 4-(3-Hydroxytetrahydropyran-3-yl)-benzol-1,3-diol.

15. Verfahren zur Synthese gemäß Anspruch 1, **dadurch gekennzeichnet, dass** X für ein Sauerstoffatom steht.

## Claims

1. A method for the synthesis of a 4-(heterocycloalkyl)-benzene-1,3-diol corresponding to the general formula (I) 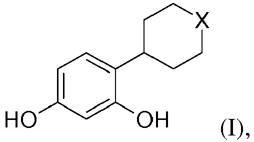 in which X represents an oxygen or a sulphur atom, wherein a compound corresponding to the general formula (7a) 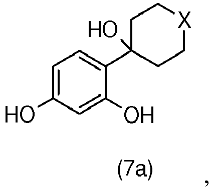 reacts with hydrogen in the presence of a palladium-based catalyst in a polar solvent when X represents an oxygen atom or with a reducing agent of the silicon hydride type and a Lewis acid in a nonpolar solvent when X represents a sulphur atom.

2. A method for the synthesis of a 4-(heterocycloalkyl)-benzene-1,3-diol corresponding to the general formula (II) 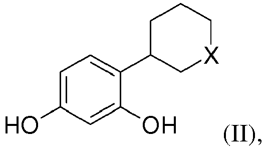 in which X represents an oxygen or a sulphur atom, wherein a compound corresponding to the general formula (7b) 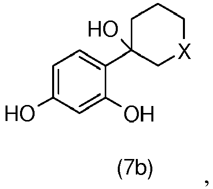 reacts with hydrogen in the presence of a palladium-based catalyst in a polar solvent when X represents an oxygen atom or with a reducing agent of the silicon hydride type and a Lewis acid in a nonpolar solvent when X represents a sulphur atom.

3. The method according to claim 1 or 2, wherein the polar solvent is selected from the group consisting of alcohols, preferably methanol, carboxylic acids, preferably acetic acid, esters, preferably ethyl acetate, ethers, preferably tetrahydrofuran, water, and a mixture thereof.

4. The method according to claim 3, wherein the alcohols are selected from the group consisting of methanol, ethanol and isopropanol.

5. The method according to claim 1 or 2, wherein the nonpolar solvent is selected from the group consisting of dichloromethane and dichloro-1,2-ethane.

6. The method according to claim 1 or 2, wherein the palladium-based catalyst is selected from the group consisting of palladium on carbon, palladium hydroxide and palladium acetate.

7. The method according to claim 1 or 2, wherein the hydrogen pressure applied is between 1 bar and 10 bar.

8. The method according to claim 1, wherein the compound corresponding to the general formula (7a) 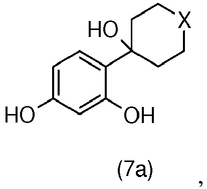 in which X represents an oxygen atom or a sulphur atom is obtained by reaction of resorcinol with a heterocycloalkanone corresponding to the general formula (3a) 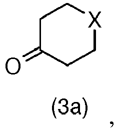 in a polar solvent and in the presence of a base.

9. The method according to claim 2, wherein the compound corresponding to the general formula (7b) 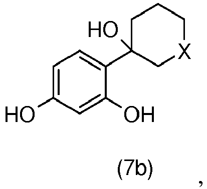 in which X represents an oxygen atom or a sulphur atom is obtained by reaction of resorcinol with a heterocycloalkanone corresponding to the general formula (3b) 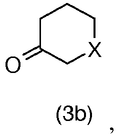 in a polar solvent and in the presence of a base.

10. The method according to claim 8 or 9, wherein the base used is selected from the group consisting of sodium hydroxide, potassium hydroxide, lithium hydroxide and metal alcoholates such as sodium methanolate or potassium tert-butylate, and preferably selected from the group consisting of sodium hydroxide and potassium hydroxide.

11. The method according to claim 8 or 9, wherein the polar solvent is selected from the group consisting of water and alcohols, preferably water, methanol, ethanol or isopropanol.

12. The method according to claim 8 or 9, wherein the resorcinol and the heterocycloalkanone (3a) or (3b) are used in a resorcinol/heterocycloalkanone molar ratio comprised between 1 and 8.

13. A compound of general formula (7a) or (7b) 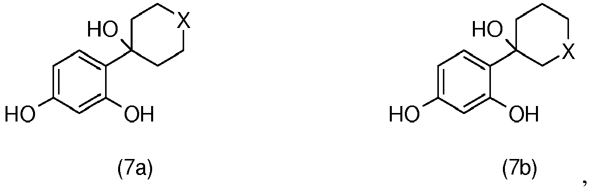 wherein X represents an oxygen atom or a sulphur atom.

14. The compound according to claim 13, wherein it is selected from the group consisting of 4-(4-hydroxy-tetrahydro-pyran-4-yl)-benzene-1,3-diol and 4-(3-hydroxy-tetrahydro-pyran-3-yl)-benzene-1,3-diol.

15. The method for the synthesis according to claim 1, wherein X represents an oxygen atom.
